# EUROPEAN PATENT SPECIFICATION

(11) **EP 1 237 949 B1**
(45) Date of publication and mention of the grant of the patent: **14.07.2004**
(21) Application number: 00968452.3
(22) Date of filing: 28.09.2000
(51) Int. Cl.: C08F 10/00, C08F 4/602

(54) **POLYMERIZATION OF OLEFINS WITH BIMETALLIC POLYMERISATION CATALYST SYSTEM**
OLEFINPOLYMERISATION MIT VERWENDUNG VON BIMETALLISCHEN KATALYSATORSYSTEMEN
POLYMERISATION D'OLEFINES A L'AIDE D'UN SYSTEME CATALYSEUR DE POLYMERISATION BIMETALLIQUE

(30) Priority: 29.09.1999 US 156681 P
(43) Date of publication of application: 11.09.2002
(73) Proprietor: E.I. DU PONT DE NEMOURS AND COMPANY, Wilmington, Delaware 19898 (US)
(72) Inventor: GUAN, Zhibin, Irvin, CA 92612 (US); WANG, Lin, Hockessin, DE 19707 (US)
(74) Representative: Carpmaels & Ransford
(86) International application number: PCT/US2000/026644
(87) International publication number: WO 2001/023444

(56) References cited:
- WO-A-97/48735
- WO-A-99/50318
- MECKING S: "REACTOR BLENDING WITH EARLY/LATE TRANSITION METAL CATALYST COMBINATIONS IN ETHYLENE POLYMERIZATION" MACROMOLECULAR: RAPID COMMUNICATIONS,DE,WILEY VCH, WEINHEIM, vol. 20, no. 3, March 1999 (1999-03), pages 139-143, XP000834998 ISSN: 1022-1336

## Description

### FIELD OF THE INVENTION

Polymers with varied and useful properties may be produced in processes using at least one polymerization catalyst, and at least one oligomerization catalyst for ethylene which is a selected iron catalyst.

### TECHNICAL BACKGROUND

Polyolefins are most often prepared by polymerization processes in which a transition metal containing catalyst system is used. Oftentimes, these transition metal catalysts will copolymerize two olefins such as ethylene and an α-olefin, especially a higher α-olefin. Such copolymers have been found very useful in a large number of fields; however, the higher cost of the α-olefin is a negative for these types of polymers compared to polymers made only from cheap olefins such as ethylene or propylene.

Various reports of "simultaneous" oligomerization and polymerization of ethylene to form (in most cases) branched polyethylenes have appeared in the literature, see for instance WO90/15085, WO99/50318, US5753785, US5856610, US5686542, US5137994 and US5071927; C. Denger, et al, Makromol. Chem. Rapid Commun., vol. 12, p. 697-701 (1991); and E. A. Benham, et al., Polymer Engineering and Science, vol. 28, p. 1469-1472 (1988). None of these references specifically describes any of the processes or resulting branched polyolefins herein.

### SUMMARY OF THE INVENTION

This invention concerns a process for preparing a branched polyolefin, comprising the steps of:
(1) contacting an ethylene oligomerization catalyst and a first monomer component comprising ethylene, under conditions to oligomerize at least a portion of the ethylene to one or more even α-olefins of the general formula R²⁸CH=CH₂ wherein R²⁸ is alkyl containing an even number of carbon atoms; and
(2) contacting an active transition metal copolymerization catalyst, with a second monomer component comprising ethylene and at least a portion of the α-olefin from step (1), under conditions to copolymerize the second monomer component,
characterized in that the ethylene oligomerization catalyst comprises an active Fe complex of a ligand of the formula (I) : 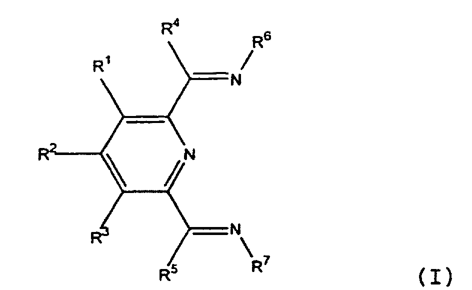 wherein:
R¹, R², R³, R⁴ and R⁵ are each independently hydrogen, hydrocarbyl, substituted hydrocarbyl, or an inert functional group, provided that any two of R¹, R² and R³ vicinal to one another, taken together may form a ring; and
R⁶ and R⁷ are aryl or substituted aryl; and
the second monomer component further comprises an odd α-olefin of the formula R¹⁸CH=CH₂ wherein R¹⁸ is alkyl containing an odd number of carbon atoms.

The two steps of the above-mentioned process may occur separately, sequentially and/or simultaneously, as described in further detail below.

### DETAILED DESCRIPTION OF THE PREFERRED EMBODIMENTS

Herein certain terms are used which are defined below.

By "hydrocarbyl" is meant a univalent radical containing only carbon and hydrogen. As examples of hydrocarbyls may be mentioned unsubstituted alkyls, cycloalkyls and aryls. If not otherwise stated, it is preferred that the hydrocarbyl groups herein contain 1 to 30 carbon atoms, and more preferably 1 to 20 carbon atoms.

By "substituted hydrocarbyl" herein is meant a hydrocarbyl group that contains one or more "inert functional groups" that are inert under the process conditions to which the compound containing these groups is subjected. The inert functional groups also do not substantially interfere with the oligomerization/polymerization process. For example, in cases in which the inert functional group may be near the complexed iron atom, such as R⁴ or R⁵ in (I) , or as a substituent on R⁴, R⁵, R⁶ or R⁷, the inert functional group should not coordinate to the iron atom more strongly than the three depicted N groups in (I) which are the desired coordinating groups - that is, the functional group should not displace one or more of the desired coordinating N groups. The hydrocarbyl may be completely substituted, as in trifluoromethyl. If not otherwise stated, it is preferred that substituted hydrocarbyl groups herein contain 1 to about 30 carbon atoms. Included in the meaning of "substituted" are heterocyclic rings.

Examples of inert functional groups include halo (fluoro. chloro, bromo and iodo), ester, keto (oxo), amino, imino, carboxyl, phosphite, phosphonite, phosphine, phosphinite, thioether, amide, nitrile, and ether. Preferred inert functional groups are halo, ester, amino, imino, carboxyl, phosphite, phosphonite, phosphine, phosphinite, thioether, and amide. Which inert functional groups are useful in which oligomerizations/polymerizations may in some cases be determined by reference to US5955555, US6103946 and WO98/30612.

By an oligomerization or polymerization "catalyst activator" is meant a compound that reacts with a transition metal compound to form an activated catalyst species. A preferred catalyst activator is an alkylaluminum compound, that is, a compound which has at least one alkyl group bound to an aluminum atom.

By "relatively noncoordinating" (or "weakly coordinating") anions are meant those anions as are generally referred to in the art in this manner, and the coordinating ability of such anions is known and has been discussed in the literature. See, for instance, W. Beck et al., Chem. Rev., vol. 88, pp. 1405-1421 (1988), and S.H. Strauss, Chem. Rev., vol. 93, pp. 927-942 (1993). Among such anions are those formed from aluminum compounds (such as those described in the immediately preceding paragraph) and X⁻ (an anion as discussed in further detail below), including (R¹⁹)₃AlX⁻, (R¹⁹)₂AlClX⁻, R¹⁹AlCl₂X⁻, and R¹⁹AlOX⁻, wherein R¹⁹ is alkyl. Other useful noncoordinating anions include BAF⁻ {BAF = tetrakis[3,5-bis(trifluoromethyl)phenyl]borace}, SbF₆⁻, PF₆⁻, and BF₄⁻, trifluoromethanesulfonate, p-toluenesulfonate, (R_{f}SO₂) ₂N⁻, and (C₆F₆)₄B⁻.

By a "primary carbon group" herein is meant a group of the formula -CH₂---, wherein the free valence --- is to any other atom, and the bond represented by the solid line is to a ring atom of an aryl or substituted aryl to which the primary carbon group is attached. Thus the free valence --- may be bonded to a hydrogen atom, a halogen atom, a carbon atom, an oxygen atom, a sulfur atom, etc. In other words, the free valence --- may be to hydrogen, hydrocarbyl, substituted hydrocarbyl or a functional group. Examples of primary carbon groups include -CH₃, -CH₂CH(CH₃)₂, -CH₂Cl, -CH₂C₆H₅, -OCH₃ and -CH₂OCH₃.

By a secondary carbon group is meant the group 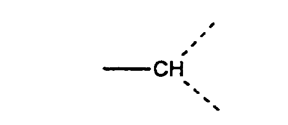 wherein the bond represented by the solid line is to a ring atom of an aryl or substituted aryl to which the secondary carbon group is attached, and both free bonds represented by the dashed lines are to an atom or atoms other than hydrogen. These atoms or groups may be the same or different. In other words the free valences represented by the dashed lines may be hydrocarbyl, substituted hydrocarbyl or inert functional groups. Examples of secondary carbon groups include -CH(CH₃)₂, -CHCl₂, -CH(C₆H₅)₂, cyclohexyl, -CH(CH₃)OCH₃, and -CH=CCH₃.

By a "tertiary carbon group" is meant a group of the formula 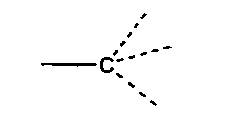 wherein the bond represented by the solid line is to a ring atom of an aryl or substituted aryl to which the tertiary carbon group is attached, and the three free bonds represented by the dashed lines are to an atom or atoms other than hydrogen. In other words, the bonds represented by the dashed lines are to hydrocarbyl, substituted hydrocarbyl or inert functional groups. Examples of tetiary carbon groups include -C(CH₃)₃, -C(C₆H₅)₃, -CC1₃, -CF₃, -C(CH₃)₂OCH₃, -C≡CH, -C(CH₃)₂CH=CH₂, aryl and substituted aryl such as phenyl and 1-adamantyl.

By "aryl" is meant a monovalent aromatic group in which the free valence is to the carbon atom of an aromatic ring. An aryl may have one or more aromatic rings which may be fused, connected by single bonds or other groups.

By "substituted aryl" is meant a monovalent aromatic group substituted as set forth in the above definition of "substituted hydrocarbyl". Similar to an aryl, a substituted aryl may have one or more aromatic rings which may be fused, connected by single bonds or other groups; however, when the substituted aryl has a heteroaromatic ring, the free valence in the substituted aryl group can be to a heteroatom (such as nitrogen) of the heteroaromatic ring instead of a carbon.

For ligand (I), preferred formulas and compounds (and for their Fe complexes also) are found in US6103946, and preferred groupings and compounds in this application are also preferred herein.

More specifically, the preferrred oligomerization catalyst is an Fe complex (Fe[II] or Fe [III]) of a ligand of the general formula (I), wherein:
R¹, R² and R³ are each independently hydrogen, hydrocarbyl, substituted hydrocarbyl or an inert functional group, provided that any two of R¹, R² and R³ vicinal to one another taken together may form a ring;
R⁴ and R⁵ are each independently hydrogen, hydrocarbyl, substituted hydrocarbyl or an inert functional group;
R⁶ and R⁷ are each independently an aryl or substituted aryl having a first ring atom bound to the imino nitrogen, provided that:
   in R⁶, a second ring atom adjacent to said first ring atom is bound to a halogen, a primary carbon group, a secondary carbon group or a tertiary carbon group; and further provided that
   in R⁶, when said second ring atom is bound to a halogen or a primary carbon group, none, one or two of the other ring atoms in R⁶ and R⁷ adjacent to said first ring atom are bound to a halogen or a primary carbon group, with the remainder of the ring atoms adjacent to said first ring atom being bound to a hydrogen atom; or
   in R⁶, when said second ring atom is bound to a secondary carbon group, none, one or two of the other ring atoms in R⁶ and R⁷ adjacent to said first ring atom are bound to a halogen, a primary carbon group or a secondary carbon group, with the remainder of the ring atoms adjacent to said first ring atom being bound to a hydrogen atom; or
   in R⁶, when said second ring atom is bound to a tertiary carbon group, none or one of the other ring atoms in R⁶ and R⁷ adjacent to said first ring atom are bound to a tertiary carbon group, with the remainder of the ring atoms adjacent to said first ring atom being bound to a hydrogen atom.

By a "first ring atom in R⁶ and R⁷ bound to an imino nitrogen atom" is meant the ring atom in these groups bound to an imino nitrogen shown in (I), for example 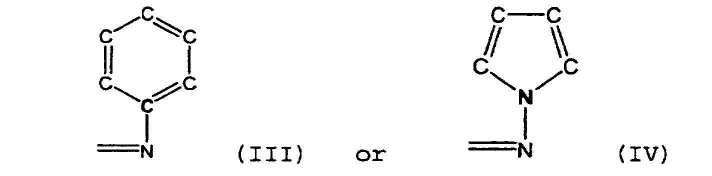 the atoms shown in the 1-position in the rings in (III) and (IV) are the first ring atoms bound to an imino carbon atom (other groups which may be substituted on the aryl groups are not shown). Ring atoms adjacent to the first ring atoms are shown, for example, in (V) and (VI), where the open valencies to these adjacent atoms are shown by dashed lines (the 2,6-positions in (V) and the 2,5-positions in (VI)).

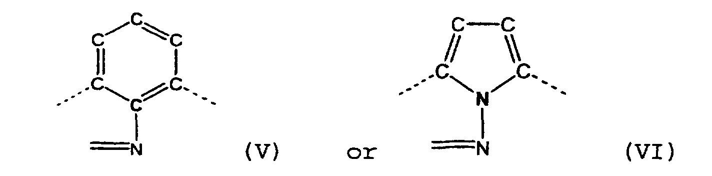

Particularly preferred is a ligand of the formula (II): 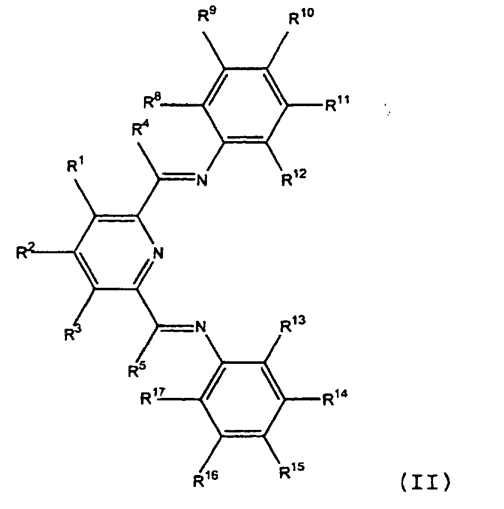 wherein:
each of R¹, R², R³, R⁴, R⁵, R⁹, R¹⁰, R¹¹, R¹⁴, R¹⁵ and R¹⁶ is independently selected from the group consisting of hydrogen, hydrocarbyl, substituted hydrocarbyl and an inert functional group; and
R⁸ is halogen, a primary carbon group, a secondary carbon group or a tertiary carbon group;
provided that:
when R⁸ is halogen or a primary carbon group none, one or two of R¹², R¹³ and R¹⁷ are independently a primary carbon group, an inert functional group or a trihalo tertiary carbon group, and the remainder of R¹², R¹³ and R¹⁷ are hydrogen;
when R⁸ is a secondary carbon group, none or one of R¹², R¹³ and R¹⁷ is a primary carbon group, a secondary carbon group, a trihalo tertiary carbon group or an inert functional group, and the remainder of R¹², R¹³ and R¹⁷ are hydrogen;
when R⁸ is a tertiary carbon group all of R¹², R¹³ and R¹⁷ are hydrogen;
any two of R¹, R² and R³ vicinal to one another, taken together may form a ring; and
any two of R⁸, R⁹, R¹⁰, R¹¹, R¹², R¹³, R¹⁴, R¹⁵, R¹⁶ and R¹⁷ vicinal to one another, taken together may form a ring.

In one preferred embodiment of ligand (II), R⁴ and R⁵ are methyl or hydrogen; and/or R¹, R², and R³ are all hydrogen; and/or R⁹, R¹⁰, R¹¹, R¹⁴, R¹⁵ and R¹⁶ are all hydrogen; and/or R¹⁷ is selected from the group consisting of methyl, ethyl, propyl isopropyl, halo and trihalomethyl; and/or R¹² is selected from the group consisting of hydrogen, methyl, ethyl, propyl, isopropyl, halo and trihalomethyl. In certain more preferred embodiments, both R¹² and R¹⁷ are methyl or ethyl. In all such cases, R⁸ is a primary carbon group, and R¹³ is hydrogen.

In specific preferred embodiments of ligand (II):
R⁴ and R⁵ are methyl; R⁹, R¹⁰, R¹¹, R¹³, R¹⁴, R¹⁵ and R¹⁶ are all hydrogen; R¹² is hydrogen or methyl; R¹⁷ is methyl; and R⁸ is a primary carbon group; or
R⁴ and R⁵ are methyl; R⁹, R¹⁰, R¹¹, R¹³, R¹⁴, R¹⁵ and R¹⁶ are all hydrogen; R¹² is hydrogen or ethyl; R¹⁷ is ethyl; and R⁸ is a primary carbon group; or
R⁴ and R⁵ are methyl; R⁹, R¹⁰, R¹¹, R¹³, R¹⁴, R¹⁵ and R¹⁶ are all hydrogen; R¹² is hydrogen or isopropyl; R¹⁷ is isopropyl; and R⁸ is a primary carbon group; or
R⁴ and R⁵ are methyl; R⁹, R¹⁰, R¹¹, R¹³, R¹⁴, R¹⁵ and R¹⁶ are all hydrogen; R¹² is hydrogen or n-propyl; R¹⁷ is n-propyl; and R⁸ is a primary carbon group; or
R⁴ and R⁵ are methyl; R⁹, R¹⁰, R¹¹, R¹³ , R¹⁴, R¹⁵ and R¹⁶ are all hydrogen; R¹² is hydrogen or chloro; R¹⁷ is chloro; and R⁸ is a primary carbon group; or
R⁴ and R⁵ are methyl; R⁹, R¹⁰, R¹¹, R¹³, R¹⁴, R¹⁵ and R¹⁶ are all hydrogen; R¹² is hydorgen or trifluoromethyl; R¹⁷ is trifluoromethyl; and R⁸ is a primary carbon group.

In another preferred embodiment of ligand (II), R¹, R², R³, R⁴, R⁵, R⁹, R¹⁰, R¹¹, R¹⁴, R¹⁵ and R¹⁶ are as just defined, and if R⁸ is a primary carbon group, R¹² and R¹⁷ are hydrogen, and R¹³ is a primary carbon group; or if R⁸ is a secondary carbon group, R¹² and R¹⁷ are hydrogen, and R¹³ is a primary carbon group or a secondary carbon group.

Also preferred is when R⁸ is a primary carbon group, preferably selected from methyl, ethyl, propyls and butyls.

US5955555, US6103946 and WO98/30612, as well as WO99/50273 (equivalent to United States Patent Application Serial No. 08/277910, filed March 29, 1999), describe synthesis of ligand (I) and its Fe complexes, and reference may be had thereto for further details.

There are many different ways of preparing active oligomerization catalysts from ligand (I) or its Fe complexes many of which are described in US5955555, US6103946 and WO98/30612, as well as WO99/50273 (equivalent to United States Patent Application Serial No. 08/277910, filed March 29, 1999), and those so described are applicable herein.

"Pure" Fe complexes may be exemplified by the formula (I)FeXₙ, wherein each X is an anion, n is 1, 2 or 3 so that the total number of negative charges on the X groups is equal to the oxidation state of the Fe in the pure Fe complex. Preferably, each X is a monovalent anion, more preferably selected from the group consisting of a halide and a nitrile, and especially a halide such as chloride or bromide.

These pure Fe complexes may in and of themselves be active oligomerization catalysts, or they may be activated (or made more active) preferably by preparation in situ by contact with a catalyst activator in a variety of methods. Generally, it has been found that the most active catalysts are those that have been contacted with a catalyst activator.

In general, details for the preparation of oligomers (sometimes referred to as α-olefins) from ethylene using the oligomerization catalysts herein can be found in US6103946, as well as B. L. Small, et. al., J. Am. Chem. Soc., vol. 120, p. 7143-7144 (1998).

Ethylene may be oligomerized by contacting a first compound W, which is a neutral Lewis acid capable of abstracting X⁻ to form WX⁻, with an iron halide complex of ligand (I) [or other X⁻ complex of (I)], provided that the anion formed is a weakly coordinating anion; or a cationic Lewis or Bronsted acid whose counterion is a weakly coordinating anion.

In those instances in which the Fe complex of (I) does not contain an alkyl, hydride, or other group which may be displaced by ethylene already bonded to the metal (i.e., X is not alkyl or hydride), a neutral Lewis acid or a cationic Lewis or Bronsted acid may also alkylate or add a hydride to the metal, i.e., causes an alkyl group or hydride to become bonded to the metal atom, or a separate compound is added to add the alkyl or hydride group.

A preferred neutral Lewis acid, which can alkylate the metal, is a selected alkyl aluminum compound, such as R²⁰₃Al, R²⁰₃AlCl, R²⁰AlCl₂ and "R²⁰AlO" (alkylaluminoxanes), wherein R²⁰ is alkyl containing 1 to 25 carbon atoms, preferably 1 to 4 carbon atoms. Suitable alkyl aluminum compounds include methylaluminoxane (which is an oligomer with the general formula [MeAlO]ₙ), (C₂H₅)₂AlCl, (C₂H₅)AlCl₂ and [(CH₃)₂CHCH₂]₃Al. Metal hydrides such as NaBH₄ may be used to bond hydride groups to the Fe.

Preferably the oligomer produced by the oligomerization catalyst is a series of compounds of the formula H₂C=CHR²⁸, wherein R²⁸ is n-alkyl containing an even number of carbon atoms. It is preferred that the series of α-olefins comprises individual α-olefins in which R¹⁸ contains 2, 4, 6, 8, 10, 12, 14, 16 and optionally higher carbon atoms. Normally, the product of the oligomerization will be a mixture of oligomers of the above formula, preferably possessing a number average molecular weight of about 600 or less, more preferably about 400 or less. Other olefins may optionally be added to the process at any point, so that they also copolymerize into the polyolefin ultimately formed.

If the olefins is made first in series with the copolymerization reaction and thus may be sampled, the olefin series being used may be analyzed, as by gas chromatography, to see if any of the above compositional limits on the olefin series are being met. If the olefin series is produced in situ simultaneously with the copolymerization reaction, it may not be possible to obtain a representative sample of the olefin series. Generally the olefin series will be produced in situ by an ethylene oligomerization catalyst which forms the requisite olefins from ethylene, and is active in the absence of the copolymerization catalyst. In this instance an oligomerization may be run in the absence of the copolymerization catalyst to produce only the series of olefins, under conditions which reasonably mimic the combined oligomerization/copolymerization. The series of olefins thus obtained is then analyzed (as by gas chromatography) to determine if it meets appropriate limitations. Typical analyses of such series of olefins may be found in US6103946. It is assumed herein that the incorporation of α-olefins into a branched polyolefin is in proportion to the relative amounts in which they are present in the polymerization process. This may not be totally correct in the event, for example, thai a volatile olefin such as 1-butene is partially "lost" co the polymerization reaction.

Oftentimes when such a series of olefins is made from ethylene a measure of the molecular weights of the olefins obtained is factor K from the Schulz-Flory theory (see for instance B. Elvers, et al., Ed. Ullmann's Encyclopedia of Industrial Chemistry, Vol. A13, VCH Verlagsgesellschaft mbH, Weinheim, 1989, p. 243-247 and 275-276. This is defined as:

K = n(Cₙ₊₂ olefin)/n(Cₙ olefin)

wherein n(Cₙ olefin) is the number of moles of olefin containing n carbon atoms, and n(Cₙ₊₂ olefin) is the number of moles of olefin containing n+2 carbon atoms, or in other words the next higher oligomer of Cₙ olefin. From this. can be determined the weight (mass) fractions of the various olefins in the resulting oligomeric reaction product mixture. The K factor is preferred to be in the range of 0.55 to 0.90, more preferably 0. 65 to 0.80. The K factor may also be varied by changing oligomerization conditions and/or the oligomerization catalyst, see for instance US6103946. By analyzing the branching pattern of the polymer produced one can roughly back calculate the K factor for the oligomerization to α-olefin, although there are possible errors (see below),

The copolymerization catalyst is a catalyst chemically different from the oligomerization catalyst, and which is capable of copolymerizing ethylene and various α-olefins, such as any one or combination of a number of well-known Ziegler-Natta-type or metallocene-type catalysts. Other suitable types of copolymerization catalysts include transition metal complexes of amidimidates and certain iron or cobalt complexes of (I).

The synthesis of the branched copolymers in accordance with the present invention herein can produce unique polymers because of the nature of the two catalysts. In preferred embodiments (discussed below) the oligomerization and copolymerization are performed simultaneously, and/or the oligomerization and copolymerization occur at comparable rates, to prepare various unique copolymers.

In one preferred form the process is carried out in the gas phase, especially when crystalline polymers are desired. It is believed that in many cases in gas phase polymerization when both catalysts are present in the same particle on which polymerization is taking place (for example originally a supported catalyst), the α-olefin is especially efficiently used (polymerized into the resulting polymer). The process may also be carried out in liquid slurry or solution, particularly where amophous or less crystalline copolymers are desired.

As just indicated, the process in accordance with the present invention can produce novel polyethylenes. By "polyethylene" in this instance is meant a polymer produced in a polymerization in which ethylene is the source of at least 50 mole percent, more preferably at least 80 mole percent and more preferably at least 90 mole percent of the repeat units in the polymer. However it is understood that the polymer produced is not made by the direct polymerization of ethylene alone, but by the copolymerization of ethylene and even α-olefins which are produced in situ, and with the odd α-olefin(s) containing (one or more) odd numbers of carbon atoms. The polymer produced usually contains -R¹⁸ branches present, in other words a polymer containing branches having odd numbers of carbon atoms. The amount of these odd number branches will be related to the amount of odd α-olefin in the polymerization mixture.

In addition, the polymer produced will contain branches of the formula (excluding end groups) -(CH₂CH₂)ₙH wherein n is 1 or more. Preferably, the polymer contains 1 to 100, more preferably 1 to 30, of these branches per 1000 methylene atoms. Normally there will be branches with a range of "n" in the polymer. The amount of these branches (as measured by total methyl groups) in the polymer preferably ranges from 2 to 200, especially preferably 5 to 175, more preferably 10 to 150, and especially preferably 20 to 150 branches per 1000 methylene groups in the polymer (for the method of measurement and calculation, see US5880241). Another preferable range for these branches is 50 to 200 methyl groups per 1000 methylene carbon atoms. It is also preferable (either alone or in combination with the other preferable features above) that in these branched polymers there are at least 2 branches each of ethyl and n-hexyl or longer and at least one n-butyl per 1000 methylene groups, more preferably at least 4 branches each of ethyl and n-hexyl or longer and at least 2 n-butyl branches per 1000 methylene groups, and especially preferably at least 10 branches each of ethyl and n-hexyl or longer and at least 5 n-butyl branches per 1000 methylene groups. It is also preferred that there are more ethyl branches than butyl branches in this polyethylene.

In combination with any of the above branching patterns, the polymer preferably also has 1 to 100, more preferably 2 to 20, -R¹⁸ branches per 1000 methylene groups. Also preferably, the -R¹⁸ branches are methyl (from propene) and/or propyl (from 1-pentene) branches.

Conditions for such polymerizations, particularly for the oligomerization catalyst, are found in US6103946. Briefly, the temperature at which the polymerization is carried out is -100°C to +200°C preferably -20°C to +80°C. The polymerization pressure which is used with ethylene is not critical, atmospheric pressure to about 275 MPa, or more, being a suitable range. These polymerizations may be batch, semi-batch or continuous processes, and may be carried out in liquid medium or the gas phase.

In the polymerizations in accordance with the present invention, the resulting polymer preferably has an average degree of polymerization of at lease 50, more preferably at least 200, and especially preferably at least 400.

Many types of catalysts are useful as the copolymerization catalyst. For instance so-called Ziegler-Natta and/or metallocene-type catalysts may be used. These types of catalysts are well known in the polyolefin field, see for instance Angew. Chem., Int. Ed. Engl., vol. 34, p. 1143-1170 (1995), EP-A-0416815 and US5198401 for information about metallocene-type catalyses; and J. Boor Jr., Ziegler-Natta Catalysts and Polymerizations, Academic Press, New York, 1979 for information about Ziegler-Natta-type catalysts. Many of the useful polymerization conditions for these types of catalysts and the oligomerization catalyst coincide, so conditions for the process are easily accessible. Oftentimes a "cocatalyst" or "activator" is needed for metallocene or Ziegler-Natta-type polymerizations, much as W is sometimes needed for the oligomerization catalyst. In many instances the same compound, such as an alkylaluminum compound, may be used for these purposes for both types of catalysts.

Suitable catalysts for the copolymerization catalyst also include metallocene-type catalysts, as described in US5324800 and EP-A-0129368; particularly advantageous are bridged bis-indenyl metallocenes, for instance as described in US5145819 and EP-A-0485823. Another class of suitable catalysts comprises the well-known constrained geometry catalysts, as described in EP-A-0416815, EP-A-0420436, EP-A-0671404, EP-A-0643066 WO91/04257. Also the class of transition metal complexes described in, for example, WO98/30609, US5880241, US5955555, US6060569 and US5714556 can be used. Metallocene catalysts already known for the copolymerization of active nonconjugated dienes are described in US5229478, WO88/04674, WO99/18135 and WO99/01460, and references described therein.

All the catalysts herein may be "heterogenized" (to form a polymerization catalyst component, for instance) by coating or otherwise attaching them to solid supports, such as silica or alumina. Where an active catalyst species is formed by reaction with a compound such as an alkylaluminum compound, a support on which the alkylaluminum compound is first coated or otherwise attached is contacted with the transition metal compounds (or their precursors) to form a catalyst system in which the active polymerization catalysts are "attached" to the solid support. These supported catalysts may be used in polymerizations in organic liquids. They may also be used in so-called gas phase polymerizations in which the olefin(s) being polymerized are added to the polymerization as gases and no liquid supporting phase is present. The transition metal compounds may also be coated onto a support such as a polyolefin (polyethylene, polypropylene, etc.) support, optionally along with other needed catalyst components such as one or more alkylaluminum compounds.

The oligomers made by the oligomerization catalyst and the polymer made by the polymerization catalyst may be made in sequence, i.e., the oligomerization followed by the polymerization, as by using two vessels in series. For example, ethylene can be oligomerized in a first reactor in the presence of the oligomerization catalyst to produce an oligomer mixture, which is then transferred to a second reactor with odd α-olefin (to the extent not already present in the first monomer mixture) and additional ethylene/α-olefin feed (to the extent necessary), and copolymerization catalyst, in the amounts and under polymerization conditions required for the desired end product.

However it is preferred to carry out the entire process in the same vessel(s), i.e., carrying out steps (1) and (2) sequentially or simultaneously. This is possible because in most instances the oligomerzation/polymerization catalysts and conditions are compatible with each other.

One such preferred process is to contact ethylene and the oligomerization catalyst for a period of time sufficient to oligomerize a portion of the ethylene to α-olefins, after which the copolymerization catalyst is added to the vessel. The odd α-olefin, additional ethylene as needed, and other α-olefins as desired, can be added at any stage during the process.

Another preferred process is to add all components to the vessel at the same time - ethylene, odd α-olefin, oligomerization catalyst and copolymerization catalyst - and conduct the oligomerization/copolymerization simultaneously. In this case, the amount of branching due to incorporation of the α-olefins (both odd and even) in the polymer can be controlled by the ratio of oligomerization catalyst to copolymerization catalyst. The higher the proportion of oligomerization catalyst the higher the amount of branching in the resulting copolymer.

In all of these processes, it preferred to use essentially only ethylene and the odd α-olefin as monomers added into the process. Of course, other monomers/oligomers will be generated in situ and incorporated into the final copolymer, but the only monomers required to operate the process and generate the products are ethylene and the odd α-olefin.

A particularly preferred aspect of the process utilizes ethylene and the odd α-olefin as the sole added monomers, with the even α-olefins being incorporated into the final copolymer solely as a result of the in situ oligomerization of ethylene.

In the Examples, all pressures are gauge pressures.

In the Examples PMAO-IP is a form of methylaluminoxane which stays in solution in toluene, and is commercially available. In the Examples all pressures are gauge pressures. In the Examples the following compounds are used:

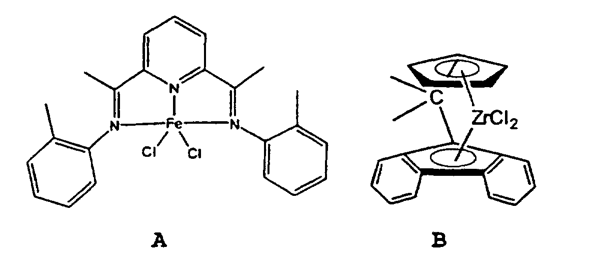

In the Examples, the following abbreviations are used:
DSC - differential scanning calorimetry
GPC - gel permeation chromatography
MAO - methylaluminoxane
MI - melt index
Mn - number average molecular weight
PE - polyethylene
RT - room temperature
TCE - tetrachloroethane

Supported catalyst used in the Examples was made by stirring a mixture of isopropylidene(cyclopentadienyl) (9-fluorenyl)zirconium dichloride (**B**, 1.0 mg), 109.2 mg (weight of solution) 0.1 wt% **A** in biphenyl, 0.35 g silica supported methylaluminoxane (18 wt% in Al) and 15 mL toluene. After shaking for 30 min, the solid was filtered, washed with 3X5 mL toluene and dried in vacuo for 1 h. It was then stored in a freezer and was used the next day.

### Example 1

A 600 mL Parr® autoclave was cleaned and was charged with 150 g of well baked NaCl. It was dried under full vacuum at 120°C for 2h. It was then charged with 690 kPa of nitrogen while it was still hot. A hot water bath was prepared by heating it to 85°C. In a drybox, 0.66 mL 13.5 wt % MAO (improved processing) toluene solution was mixed with 4 mL of toluene. It was transferred to a 5 mL syringe. This was brought out of the drybox and the solution was injected to the autoclave under positive nitrogen pressure. The mixture was stirred (600 RPM) under 690 kPa nitrogen for 30 min. Stirring was stopped. In a drybox, 120 mg of freshly made silica supported catalyst was mixed with 4.5 mL cyclohexane. This was transferred to a 5 mL gas tight syringe. It was brought out of the drybox. The mixture was then injected to the autoclave under positive nitrogen pressure. The mixture was then allowed to stir (600 RPM) under 690 kPa nitrogen for 15 min. Stirring was stopped. Nitrogen was released to 14 kPa. The autoclave was evacuated under full vacuum for 15 min, with stirring the last 5 min. It was recharged with 1.17 MPa nitrogen, then released to 14 kPa twice. The mixture was allowed to stir at 500 RPM. Ethylene was added (350 kPa). It was then pressured with propene up to 690 kPa total (350 kPa propene). Finally the total pressure of the reactor was adjusted to 2.41 MPa by feeding ethylene. The reactor was placed in the 85°C water bath. The mixture was allowed to stir at 80°C to 90°C for 2 h.. The autoclave was vented and the polymer isolated. ¹HNMR (TCE-d₂, 120°C): 65Me/1000CH₂. GPC(PE standard, 135°C): Mw = 31,744; Mn = 5,464; PD = 5.81. The polymer had a melting point of 124°C (13.0 J/g) based on DSC. MI = 6.3. The density was 0.891 g/mL based on IR. ¹³C NMR indicated that there were 61.7 Me/1000 CH₂, and branching distribution (per 1000 CH₂ groups) was: Me=41.3; Et=5.5; pr=0.0; Bu=3.9, Am=0.0; Hex+=11.5.

### Example 2

A 600 mL Parr® autoclave was cleaned, heated up under vacuum and then allowed to cool under nitrogen. It was then brought into a drybox. In the drybox, to a Hoke® cylinder was added 5 mL toluene and 1.0 mL MAO (13.5 wt% toluene solution). To a 20 mL vial was added 4.0 mg of **B** and 16 mL toluene. Only 2 mL of this solution was pipet transferred to the 600 mL autoclave. Then 57.2 mg of a 0.1 wt% **A** in biphenyl (solid) solution (weight of solution) was also added to the autoclave, followed by addition of 250 mL 2,2,4-trimethylpentane and 25 g 1-pentene. The autoclave was sealed. Both the Hoke® cylinder and the autoclave were brought out of the drybox. The autoclave was assembled to a high pressure line. The Hoke® cylinder was then connected to the autoclave. The autoclave was pressured up with nitrogen. The autoclave was heated to 85°C, and nitrogen was released to 21 kPa. It was then pressurized up with 2X to 690 kPa ethylene, venting each time and finally pressurized to 830 kPa ethylene with stirring. The MAO solution was added from the Hoke® cylinder at slightly higher pressure. The ethylene pressure of the reactor was then adjusted to 1.24 MPa. The reaction mixture was allowed to stir at 88°C-99°C for 60 min. The heating source was removed. Ethylene was vented to about 210 kPa. The autoclave was back filled with 1.38 MPa nitrogen and was then vented to 210 kPa. This was repeated. The reaction mixture was then cooled to RT. The reaction mixture was slowly poured into 400 mL methanol, followed by addition of 6 mL cone. HC1. After stirring at RT for 25 min, polymer was filtered, washed with methanol six times and dried in vacuo. White polymer (24.87 g) was obtained.

## Claims

1. A process for preparing a branched polyolefin, comprising the steps of:
(1) contacting an ethylene oligomerization catalyst and a first monomer component comprising ethylene, under conditions to oligomerize at least a portion of the ethylene to one or more even α-olefins of the general formula R²⁸CH=CH₂ wherein R²⁸ is alkyl containing an even number of carbon atoms; and
(2) contacting an active transition metal copolymerization catalyst, with a second monomer component comprising ethylene and at least a portion of the α-olefin from step (1), under conditions to copolymerize the second monomer component,
**characterized in that** the ethylene oligomerization catalyst comprises an active Fe complex of a ligand of the formula (I) : 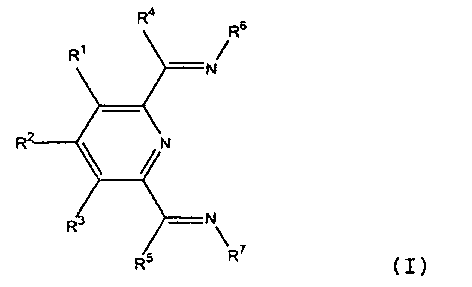 wherein:
R¹, R², R³, R⁴ and R⁵ are each independently hydrogen, hydrocarbyl, substituted hydrocarbyl, or an inert functional group, provided that any two of R¹, R² and R³ vicinal to one another, taken together may form a ring; and
R⁶ and R⁷ are aryl or substituted aryl; and
the second monomer component further comprises an odd α-olefin of the formula R¹⁸CH=CH₂ wherein R¹⁸ is alkyl containing an odd number of carbon atoms.

2. The process as recited in claim 1, **characterized in that** the oligomerization catalyst is an Fe complex of a ligand of the general formula (I), wherein:
R¹, R² and R³ are each independently hydrogen, hydrocarbyl, substituted hydrocarbyl or an inert functional group, provided that any two of R¹, R² and R³ vicinal to one another taken together may form a ring;
R⁴ and R⁵ are each independently hydrogen, hydrocarbyl, substituted hydrocarbyl or an inert functional group;
R⁶ and R⁷ are each independently an aryl or substituted aryl having a first ring atom bound to the imino nitrogen, provided that:
in R⁶, a second ring atom adjacent to said first ring atom is bound to a halogen, a primary carbon group, a secondary carbon group or a tertiary carbon group; and further provided that
in R⁶, when said second ring atom is bound to a halogen or a primary carbon group, none, one or two of the other ring atoms in R⁶ and R⁷ adjacent to said first ring atom are bound to a halogen or a primary carbon group, with the remainder of the ring atoms adjacent to said first ring atom being bound to a hydrogen atom; or
in R⁶, when said second ring atom is bound to a secondary carbon group, none, one or two of the other ring atoms in R⁶ and R⁷ adjacent to said first ring atom are bound to a halogen, a primary carbon group or a secondary carbon group, with the remainder of the ring atoms adjacent to said first ring atom being bound to a hydrogen atom; or
in R⁶, when said second ring atom is bound to a tertiary carbon group, none or one of the other ring atoms in R⁶ and R⁷ adjacent to said first ring atom are bound to a tertiary carbon group, with the remainder of the ring atoms adjacent to said first ring atom being bound to a hydrogen atom.

3. The process a recited in claim 2, **characterized in that** the oligomerization catalyst comprises an active Fe complex of a ligand of the formula (II): 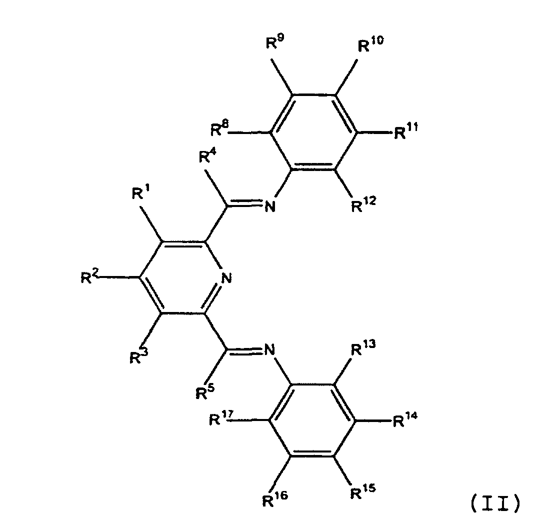 wherein:
each of R¹, R², R³, R⁴, R⁵, R⁹, R¹⁰, R¹¹, R¹⁴, R¹⁵ and R¹⁶ is independently selected from the group consisting of hydrogen, hydrocarbyl, substituted hydrocarbyl and an inert functional group; and
R⁸ is halogen, a primary carbon group, a secondary carbon group or a tertiary carbon group;
provided that:
when R⁸ is halogen or a primary carbon group none, one or two of R¹², R¹³ and R¹⁷ are independently a primary carbon group, an inert functional group or a trihalo tertiary carbon group, and the remainder of R¹², R¹³ and R¹⁷ are hydrogen;
when R⁸ is a secondary carbon group, none or one of R¹², R¹³ and R¹⁷ is a primary carbon group, a secondary carbon group, a trihalo tertiary carbon group or an inert functional group, and the remainder of R¹², R¹³ and R¹⁷ are hydrogen;
when R⁸ is a tertiary carbon group all of R¹², R¹³ and R¹⁷ are hydrogen;
any two of R¹, R² and R³ vicinal to one another, taken together may form a ring; and
any two of R⁸, R⁹, R¹⁰, R¹¹, R¹², R¹³, R¹⁴, R¹⁵, R¹⁶ and R¹⁷ vicinal to one another, taken together may form a ring.

4. The process as recited in claim 1, **characterized in that** the copolymerization catalyst is a metallocene-type catalyst.

5. The process as recited in claim 2, **characterized in that** the copolymerization catalyst is a metallocene-type catalyst.

6. The process as recited in claim 1, **characterized in that** steps (1) and (2) are conducted sequentially or simultaneously in the same reactor vessel.

7. The process as recited in claim 6, **characterized in that** steps (1) and (2) are conducted simultaneously in the same reactor vessel.

8. The process as recited in claim 7, **characterized in that** the oligomerization and copolymerization occur at comparable rates.

9. The process as recited in claim 1, **characterized in that** the odd α-olefin is selected from the group consisting of propene and pentene.

10. The process as recited in claim 1, **characterized in that** the first monomer component consists essentially of ethylene and optionally an odd α-olefin, and the second monomer component consists essentially of ethylene, at least a portion of the α-olefins from step (1) and the odd α-olefin.

11. The process as recited in claim 5, **characterized in that** steps (1) and (2) are conducted simultaneously in the same reactor vessel; the oligomerization and copolymerization catalysts are supported; the process is carried out in the gas phase; the oligomerization and copolymerization occur at comparable rates; the first monomer component consists essentially of ethylene and optionally an odd α-olefin; and the second monomer component consists essentially of ethylene, at least a portion of the α-olefins from step (1) and the odd α-olefin.

12. The process as recited in any one of claims 1-11, **characterized in that** the oligomerization and copolymerization catalysts are supported.

13. The process as recited in claim 12 carried out in the gas phase.

## Patentansprüche

1. Verfahren zur Herstellung eines verzweigten Polyolefins, umfassend die Schritte:
(1) Inkontaktbringen eines Ethylen-Oligomerisationskatalysators und einer ersten Monomerkomponente, umfassend Ethylen, unter Bedingungen, um zumindest einen Teil des Ethylens zu einem oder mehreren geradzahligen α-Olefinen der allgemeinen Formel R²⁸CH=CH₂ zu oligomerisieren, wobei R²⁸ Alkyl, enthaltend eine geradzahlige Anzahl von Kohlenstoffatomen, ist; und
(2) Inkontaktbringen eines aktiven Übergangsmetall-Copolymerisationskatalysators mit einer zweiten Monomerkomponente, umfassend Ethylen und zumindest einen Teil des α-Olefins von Schritt (1), unter Bedingungen, um die zweite Monomerkomponente zu copolymerisieren,
**dadurch gekennzeichnet, daß** der Ethylen-Oligomerisationskatalysator einen aktiven Fe-Komplex eines Liganden der Formel (I): 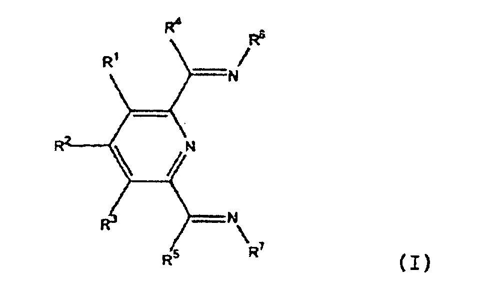 umfaßt, wobei
R¹, R², R³, R⁴ und R⁵ jeweils unabhängig Wasserstoff, Hydrocarbyl, substituiertes Hydrocarbyl oder eine inerte funktionelle Gruppe sind, mit der Maßgabe, daß alle zwei von R¹, R² und R³, die zueinander benachbart sind, zusammengenommen einen Ring bilden können;
und
R⁶ und R⁷ Aryl oder substituiertes Aryl sind; und
die zweite Monomerkomponente weiterhin ein ungeradzahliges α-Olefin der Formel R¹⁸CH=CH₂ umfaßt, wobei R¹⁸ Alkyl, enthaltend eine ungeradzahlige Anzahl von Kohlenstoffatomen, ist.

2. Verfahren nach Anspruch 1, **dadurch gekennzeichnet, daß** der Oligomerisationskatalysator ein Fe-Komplex eines Liganden der allgemeinen Formel (I) ist, wobei:
R¹, R² und R³ jeweils unabhängig Wasserstoff, Hydrocarbyl, substituiertes Hydrocarbyl oder eine inerte funktionelle Gruppe sind, mit der Maßgabe, daß alle zwei von R¹, R² und R³ die zueinander benachbart sind, zusammengenommen einen Ring bilden können;
R⁴ und R⁵ jeweils unabhängig Wasserstoff, Hydrocarbyl, substituiertes Hydrocarbyl oder eine inerte funktionelle Gruppe sind;
R⁶ und R⁷ jeweils unabhängig ein Aryl oder substituiertes Aryl sind, die ein erstes Ringatom, gebunden an den Iminostickstoff, aufweisen, mit der Maßgabe, daß:
m R⁶ ein zweites Ringatom, angrenzend an das erste Ringatom, an ein Halogen, eine primäre Kohlenstoffgruppe, eine sekundäre Kohlenstoffgruppe oder eine tertiäre Kohlenstoffgruppe gebunden ist; und weiterhin mit der Maßgabe, daß
in R⁶, wenn das zweite Ringatom an ein Halogen oder eine primäre Kohlenstoffgruppe gebunden ist, keines, eines oder zwei der anderen Ringatome in R⁶ und R⁷, angrenzend an das erste Ringatom, an ein Halogen oder eine primäre Kohlenstoffgruppe gebunden sind, wobei der Rest der Ringatome, angrenzend an das erste Ringatom, an ein Wasserstoffatom gebunden ist; oder
in R⁶, wenn das zweite Ringatom an eine sekundäre Kohlenstoffgruppe gebunden ist, keines, eines oder zwei der anderen Ringatome in R⁶ und R⁷, angrenzend an das erste Ringatom, an ein Halogen, eine primäre Kohlenstoffgruppe oder eine sekundäre Kohlenstoffgruppe gebunden sind, wobei der Rest der Ringatome, angrenzend an das erste Ringatom, an ein Wasserstoffatom gebunden ist; oder
in R⁶, wenn das zweite Ringatom an eine tertiäre Kohlenstoffgruppe gebunden ist, keines oder eines der anderen Ringatome in R⁶ und R⁷, angrenzend an das erste Ringatom, an eine tertiäre Kohlenstoffgruppe gebunden ist, wobei der Rest der Ringatome, angrenzend an das erste Ringatom, an ein Wasserstoffatom gebunden ist.

3. Verfahren nach Anspruch 2, **dadurch gekennzeichnet, daß** der Oligomerisationskatalysator einen aktiven Fe-Komplex eines Liganden der Formel (II): 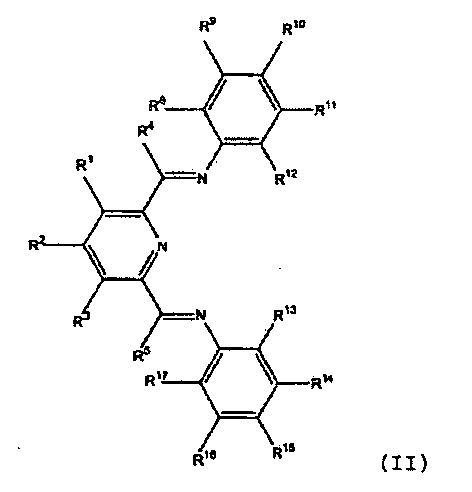 umfaßt, wobei:
jedes von R¹, R², R³, R⁴, R⁵, R⁹, R¹⁰, R¹¹, R¹⁴, R¹⁵ und R¹⁶ unabhängig aus der Gruppe, bestehend aus Wasserstoff, Hydrocarbyl, substituiertem Hydrocarbyl und einer inerten funktionellen Gruppe, ausgewählt ist; und
R⁸ Halogen, eine primäre Kohlenstoffgruppe, eine sekundäre Kohlenstoffgruppe oder eine tertiäre Kohlenstoffgruppe ist;
mit der Maßgabe, daß:
wenn R⁸ Halogen oder eine primäre Kohlenstoffgruppe ist, keines, eines oder zwei von R¹², R¹³ und R¹⁷ unabhängig eine primäre Kohlenstoffgruppe, eine inerte funktionelle Gruppe oder eine tertiäre Trihalogenkohlenstoffgruppe sind und der Rest von R¹², R¹³ und R¹⁷ Wasserstoff ist;
wenn R⁸ eine sekundäre Kohlenstoffgruppe ist, keines oder eines von R¹², R¹³ und R¹⁷ eine primäre Kohlenstoffgruppe, eine sekundäre Kohlenstoffgruppe, eine tertiäre Trihalogenkohlenstoffgruppe oder eine inerte funktionelle Gruppe ist und der Rest von R¹², R¹³ und R¹⁷ Wasserstoff ist;
wenn R⁸ eine tertiäre Kohlenstoffgruppe ist, alle von R¹², R¹³ und R¹⁷ Wasserstoff sind;
alle zwei von R¹, R² und R³, die zueinander benachbart sind, zusammengenommen einen Ring bilden können; und
alle zwei von R⁸, R⁹, R¹⁰, R¹¹, R¹², R¹³, R¹⁴, R¹⁵, R¹⁶ und R¹⁷, die zueinander benachbart sind, zusammengenommen einen Ring bilden können.

4. Verfahren nach Anspruch 1, **dadurch gekennzeichnet, daß** der Copolymerisationskatalysator ein Katalysator vom Metallocen-Typ ist.

5. Verfahren nach Anspruch 2, **dadurch gekennzeichnet, daß** der Copolymerisationskatalysator ein Katalysator vom Metallocen-Typ ist.

6. Verfahren nach Anspruch 1, **dadurch gekennzeichnet, daß** die Schritte (1) und (2) nacheinander oder gleichzeitig in demselben Reaktorgefäß durchgeführt werden.

7. Verfahren nach Anspruch 6, **dadurch gekennzeichnet, daß** die Schritte (1) und (2) gleichzeitig in demselben Reaktorgefäß durchgeführt werden.

8. Verfahren nach Anspruch 7, **dadurch gekennzeichnet, daß** die Oligomerisation und Copolymerisation mit vergleichbaren Geschwindigkeiten erfolgen.

9. Verfahren nach Anspruch 1, **dadurch gekennzeichnet, daß** das ungeradzahlige α-Olefin aus der Gruppe, bestehend aus Propen und Penten, ausgewählt ist.

10. Verfahren nach Anspruch 1, **dadurch gekennzeichnet, daß** die erste Monomerkomponente im wesentlichen aus Ethylen und gegebenenfalls einem ungeradzahligen α-Olefin besteht und die zweite Monomerkomponente im wesentlichen aus Ethylen, zumindest einem Teil der α-Olefine von Schritt (1) und dem ungeradzahligen α-Olefin besteht.

11. Verfahren nach Anspruch 5, **dadurch gekennzeichnet, daß** die Schritte (1) und (2) gleichzeitig in demselben Reaktorgefäß durchgeführt werden; die Oligomerisations- und Copolymerisationskatalysatoren geträgert sind; das Verfahren in der Gasphase ausgeführt wird; die Oligomerisation und Copolymerisation mit vergleichbaren Geschwindigkeiten erfolgen; die erste Monomerkomponente im wesentlichen aus Ethylen und gegebenenfalls einem ungeradzahligen α-Olefin besteht; und die zweite Monomerkomponente im wesentlichen aus Ethylen, zumindest einem Teil der α-Olefine von Schritt (1) und dem ungeradzahligen α-Olefin besteht.

12. Verfahren nach einem der Ansprüche 1-11, **dadurch gekennzeichnet, daß** die Oligomerisations- und Copolymerisationskatalysatoren geträgert sind.

13. Verfahren nach Anspruch 12, ausgeführt in der Gasphase.

## Revendications

1. Procédé de préparation d'une polyoléfine ramifiée, comprenant les étapes consistant à:
(1) mettre en contact un catalyseur d'oligomérisation d'éthylène et un premier composant monomère comprenant l'éthylène, dans des conditions permettant d'oligomériser au moins une partie de l'éthylène en une ou plusieurs α-oléfine(s) paire(s) de formule générale R²⁸CH=CH₂ dans laquelle R²⁸ est un alkyle contenant un nombre pair d'atomes de carbone; et
(2) mettre en contact un catalyseur de copolymérisation à base de métal de transition actif, avec un second composant monomère comprenant l'éthylène et au moins une partie de l'α-oléfine de l'étape (1), dans des conditions permettant de copolymériser le second composant monomère,
**caractérisé en ce que** le catalyseur d'oligomérisation à base d'éthylène comprend un complexe de Fe actif d'un ligand de formule (I): 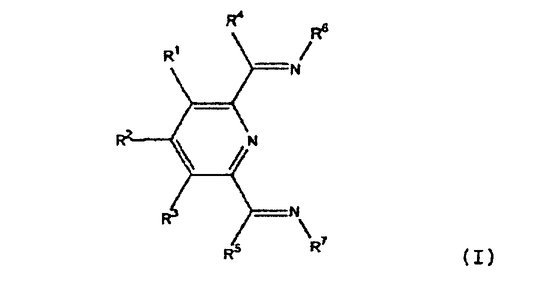 dans laquelle:
R¹, R², R³, R⁴ et R⁵ sont chacun indépendamment l'hydrogène, un hydrocarbyle, un hydrocarbyle substitué, ou un groupe fonctionnel inerte, à condition que deux quelconques de R¹, R² et R³ vicinaux l'un par rapport à l'autre, pris ensemble puissent former un cycle; et
R⁶ et R⁷ sont un aryle ou un aryle substitué; et
le second composant monomère comprend en outre une α-oléfine impaire de formule R¹⁸CH=CH₂ dans laquelle R¹⁸ est un alkyle contenant un nombre impair d'atomes de carbone.

2. Procédé tel qu'énoncé dans la revendication 1, **caractérisé en ce que** le catalyseur d'oligomérisation est un complexe de Fe d'un ligand de formule générale (I), dans laquelle:
R¹, R² et R³ sont chacun indépendamment l'hydrogène, un hydrocarbyle, un hydrocarbyle substitué ou un groupe fonctionnel inerte, à condition que deux quelconques de R¹, R² et R³ vicinaux l'un par rapport à l'autre pris ensemble puissent former un cycle;
R⁴ et R⁵ sont chacun indépendamment l'hydrogène, un hydrocarbyle, un hydrocarbyle substitué ou un groupe fonctionnel inerte;
R⁶ et R⁷ sont chacun indépendamment un aryle ou un aryle substitué ayant un premier atome cyclique lié à l'azote imino, à condition que:
dans R⁶, un second atome cyclique adjacent audit premier atome cyclique soit lié à un halogène, un groupe carbone primaire, un groupe carbone secondaire ou à un groupe carbone tertiaire; et en outre à condition que
dans R⁶, lorsque ledit second atome cyclique est lié à un halogène ou à un groupe carbone primaire, aucun, un ou deux des autres atomes cycliques dans R⁶ et R⁷ adjacents audit premier atome cyclique soient liés à un halogène ou à un groupe carbone primaire, avec le reste des atomes cycliques adjacents audit premier atome cyclique étant lié à un atome d'hydrogène; ou
dans R⁶, lorsque ledit second atome cyclique est lié à un groupe carbone secondaire, aucun, un ou deux des autres atomes cycliques dans R⁶ et R⁷ adjacents audit premier atome cyclique soient liés à un halogène, un groupe carbone primaire ou un groupe carbone secondaire, avec le reste des atomes cycliques adjacents audit premier atome cyclique étant lié à un atome d'hydrogène; ou
dans R⁶, lorsque ledit second atome cyclique est lié à un groupe carbone tertiaire, aucun ou l'un des autres atomes cycliques dans R⁶ et R⁷ adjacents audit premier atome cyclique soient liés à un groupe carbone tertiaire, avec le reste des atomes cycliques adjacents audit premier atome cyclique étant lié à un atome d'hydrogène.

3. Procédé tel qu'énoncé dans la revendication 2, **caractérisé en ce que** le catalyseur d'oligomérisation comprend un complexe de Fe actif d'un ligand de formule (II): 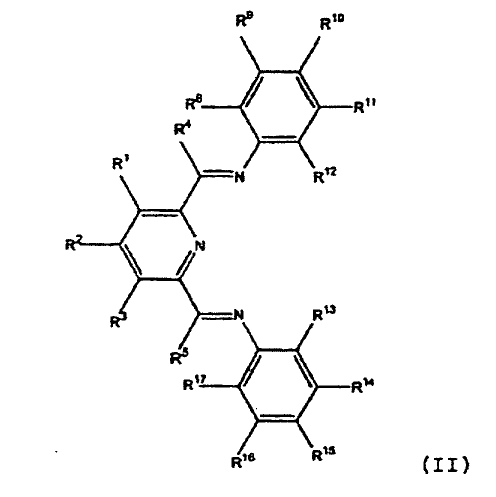 dans laquelle:
chacun de R¹, R², R³, R⁴, R⁵, R⁹, R¹⁰, R¹¹, R¹⁴, R¹⁵ et R¹⁶ est indépendamment choisi au sein du groupe constitué de l'hydrogène, d'un hydrocarbyle, d'un hydrocarbyle substitué et d'un groupe fonctionnel inerte; et
R⁸ est un halogène, un groupe carbone primaire, un groupe carbone secondaire ou un groupe carbone tertiaire;
à condition que:
lorsque R⁸ est un halogène ou un groupe carbone primaire aucun, un ou deux de R¹², R¹³ et R¹⁷ soient indépendamment un groupe carbone primaire, un groupe fonctionnel inerte ou un groupe carbone tertiaire trihalogéné, et le reste de R¹², R¹³ et R¹⁷ soient l'hydrogène;
lorsque R⁸ est un groupe carbone secondaire, aucun ou l'un de R¹², R¹³ et R¹⁷ soit un groupe carbone primaire, un groupe carbone secondaire, un groupe carbone tertiaire trihalogéné ou un groupe fonctionnel inerte, et le reste de R¹², R¹³ et R¹⁷ soient l'hydrogène;
lorsque R⁸ est un groupe carbone tertiaire tous les R¹², R¹³ et R¹⁷ soient l'hydrogène;
deux quelconques de R¹, R² et R³ vicinaux l'un par rapport à l'autre, pris ensemble puissent former un cycle; et
deux quelconques de R⁸, R⁹, R¹⁰, R¹¹, R¹², R¹³, R¹⁴, R¹⁵, R¹⁶ et R¹⁷ vicinaux l'un par rapport à l'autre, pris ensemble puissent former un cycle.

4. Procédé tel qu'énoncé dans la revendication 1, **caractérisé en ce que** le catalyseur de copolymérisation est un catalyseur de type métallocène.

5. Procédé tel qu'énoncé dans la revendication 2, **caractérisé en ce que** le catalyseur de copolymérisation est un catalyseur de type métallocène.

6. Procédé tel qu'énoncé dans la revendication 1, **caractérisé en ce que** les étapes (1) et (2) sont conduites de manière séquentielle ou simultanée dans le même récipient de réaction.

7. Procédé tel qu'énoncé dans la revendication 6, **caractérisé en ce que** les étapes (1) et (2) sont conduites simultanément dans le même récipient de réaction.

8. Procédé tel qu'énoncé dans la revendication 7, **caractérisé en ce que** l'oligomérisation et la copolymérisation se produisent à des vitesses comparables.

9. Procédé tel qu'énoncé dans la revendication 1, **caractérisé en ce que** l'α-oléfine impaire est choisie au sein du groupe constitué du propène et du pentène.

10. Procédé tel qu'énoncé dans la revendication 1, **caractérisé en ce que** le premier composant monomère est constitué essentiellement d'éthylène et éventuellement d'une α-oléfine impaire, et le second composant monomère est constitué essentiellement d'éthylène, d'au moins une partie des α-oléfines provenant de l'étape (1) et de l'α-oléfine impaire.

11. Procédé tel qu'énoncé dans la revendication 5, **caractérisé en ce que** les étapes (1) et (2) sont conduites simultanément dans le même récipient de réaction; les catalyseurs d'oligomérisation et de copolymérisation sont supportés; le procédé est effectué en phase gazeuse; l'oligomérisation et la copolymérisation se produisent à des vitesses comparables; le premier composant monomère est essentiellement constitué d'éthylène et éventuellement d'une α-oléfine impaire; et le second composant monomère est constitué essentiellement d'éthylène, d'au moins une partie des α-oléfines provenant de l'étape (1) et de l'α-oléfine impaire.

12. Procédé tel qu'énoncé dans l'une quelconque des revendications 1 à 11, **caractérisé en ce que** les catalyseurs d'oligomérisation et de copolymérisation sont supportés.

13. Procédé tel qu'énoncé dans la revendication 12 effectué en phase gazeuse.
